# EUROPEAN PATENT APPLICATION

(11) **EP 2 305 283 A1**
(43) Date of publication of application: **06.04.2011**
(21) Application number: 09012144.3
(22) Date of filing: 24.09.2009
(51) Int. Cl.: A61K 38/10, A61K 31/23, A61P 29/00

(54) **Pharmaceutical compositions for treating dysregulated inflammatory diseases**

(71) Applicant: Helmholtz-Zentrum für Infektionsforschung GmbH, 38124 Braunschweig (DE); Medizinische Hochschule Hannover, 30625 Hannover (DE)
(72) Inventor: Mühlradt, Peter, 38119 Braunschweig (DE); Barkhausen, Tanja, 27336 Frankenfeld (DE); Tschernig, Thomas, 66482 Zweibrücken (DE)
(74) Representative: Kröncke, Rolf

(57) **Abstract**

In general, the present invention relates to a new use of lipopeptides or lipoprotein molecules, namely, for treating dysregulated inflammatory diseases. In particular, the present invention relates to pharmaceutical compositions for use in treating dysregulated inflammatory diseases including SIRS, MODS and sepsis. For example, the MALP-2 molecule allows to treat sepsis.

## Description

In general, the present invention relates to a new use of lipopeptides or lipoprotein molecules, namely, for treating dysregulated inflammatory diseases. In particular, the present invention relates to pharmaceutical compositions for use in treating dysregulated inflammatory diseases including SIRS, MODS and sepsis. For example, the MALP-2 molecule allows to treat SIRS or sepsis.

### Prior art

Dysregulated inflammatory diseases may occur as common complications on intensive care units. For example after polytraumatic injuries, an uncontrolled activation of the immune system may occur. These type of uncontrolled activations are known as systemic inflammatory response syndrome (SIRS) or compensatory anti-inflammatory response syndrome (CARS). SIRS is an inflammatory state affecting the whole body, frequently but not necessarily in response to an infection. Manifestation of SIRS include but are not limited to body temperatures less than 36°C or greater than 38°C, heart rate higher than 90 beats per minute, tachypnea with more than 20 breaths per minute or an arterial partial pressure of carbon dioxide of less than 4,3 kPa, white blood cell counts of < 4000 cells/mm³ or > 12000 cells/mm³ or the presence of >10 % immature neutrophils. SIRS is frequently complicated by failure of one or more organs or organ systems e.g. acute lung injury, acute kidney injury, shock or multiple organ dysfunction syndrome. When SIRS is due to an infection, it is considered as a sepsis while non-infectious causes of SIRS includes trauma, burns, pancreatitis, ischemia and hemorrhage.

CARS is used for identifying syndromes or disorders where an excessive anti-inflammatory response is observed. It is assumed that CARS represents an excessive backlash of the body with an overshooting anti-inflammatory response. As a consequence, immune paralysis or immune suppression may occur, resulting in an increased susceptibility to infection. CARS promotes the development of septic complications during etiopathology. In cases when SIRS and CARS occur in parallel, a so called mixed antagonistic response syndrome (MARS) is present.

Another form of dysregulated inflammatory disease is known under the term "multiple organ dysfunction syndrome" (MODS), previously known as multiple organ failure (MOF). Said syndrome is characterized in altered organ function in acutely ill patients, such that homeostasis can not be maintained without intervention. It usually involves two or more organ systems. The primary cause of MODS triggers an uncontrolled inflammatory response. For example in operative and non-operative patients sepsis is the most common cause. SIRS, CARS, MARS but also sepsis may ultimately progress to MODS. At present there is rarely an agent that can reverse sepsis and established organ failure, therapy is limited to supportive care. Today the administration of human recombinant activated protein C is suggested for the treatment of MODS.

Sepsis is one form of a serious medical condition of SIRS when infection or suspected infection is present. A severe form of sepsis occurs when sepsis leads to organ dysfunction, low blood pressure or insufficient blood flow to one or more organs. Sepsis can lead to septic shock, MODS and, finally, death.

A definition of SIRS, sepsis and organ failure, etc as well as guidelines for use of innovative therapies in sepsis are outlined in Bone, R. C., et al. Chest 1992, 101, 1644-1655, which is incorporated herein by reference. Furhther, this document provides guidelines for determining sepsis etc based on different parameters like temperature, heart rate, etc.

In the United States e.g. sepsis is the second-leading cause of death in non-coronary intensive-care-unit (ICU) patients, and the tenth-most-common cause of death overall according to data from the Centers for Disease Control and Prevention. Sepsis occurs in one to two percent of all hospitalisations and accounts for as much as 25 % of ICU bed utilization. It is a major cause of death in ICU worldwide with mortality rates that range from 20 % for sepsis to 40 % for severe sepsis to about 60 % for septic shock resulting in MODS.

The therapy of sepsis rests on antibiotics, surgical drainage of infected fluid collections, fluid replacement and appropriate support for organ dysfunction. Furthermore, the administration of activated protein C is suggested for therapy of sepsis, septic shock and MODS. However, administration of activated protein C reduces death of affected patients by 6.1 % only. In addition, administration of activated protein C may not be a choice in polytrauma patients due to an increased propensity for bleeding.

Hence, there is an urgent need for alternative therapeutic agents for treating the diseases, disorders or conditions mentioned above, e.g. SIRS, CARS, sepsis or MODS, that prevail in particular in intensive care units. Patients exhibiting the referenced dysregulated inflammatory diseases are at high risk for developing multi organ failure resulting in mortality rates in the range from 10 to 60 % depending on sub-collectives. It is noteworthy that the high mortality rates are not primarily caused by the invading bacteria but are rather be induced by an overwhelming reaction of the body defence system against infection.

Macrophage-activating lipopeptide-2 (MALP-2) is a component of the cell membrane of *mycoplasma fermentans* of 2 kDa molecular mass. MALP-2 has been identified as a macrophage activating lipopeptide. Subsequent studies revealed that MALP-2 is useful in the prophylaxis and treatment of various diseases as well as a potent adjuvant in vaccines. For example WO98/27110 describes MALP-2 as useful for the prophylaxis of infections and for stimulation of the immune response. Further it has been identified that said molecule may allow desensitisation of individuals against endotoxins which are regarded as being responsible for inducing gram-negative septic shock. In addition, WO 99/59610 describes the use of lipopeptides including MALP-2 for the treatment of wounds in human and animals. Moreover the use of said lipopeptides for treating lung infections and lung tumors are disclosed in WO 02/28887. For MALP-2 it has been shown in *in vitro* as well as in animal experiments that preexposure to MALP-2 can alleviate the results of endotoxins as a cause of sepsis. That is, MALP-2 can induce endotoxin tolerance. Further, it has been described that prophylactic treatment of MALP-2 may prevent sepsis (Sato et al., 2000. J. Immunol. December 15; 165 (12): 7096 - 101; Deiters et al., 2003, Infect. Immun. 71(8): 4456 - 62). It has been speculated that prophylactic treatment with MALP-2 before or during surgery may diminish the risk of sepsis and infection as well as reduce the overshooting production of harmful proinflammatory cytokines.

In support of the results above, a recent study in a murine sepsis model demonstrated that pretreatment with MALP-2 four days before sepsis induction leads to a partial protection against sepsis-related mortality (Feterowski C. et al. 2005 Int. Immuno1.17:1035 to 1046). Consequently, the general opinion on the use of MALP-2 was to administer MALP-2 or other lipopeptide molecules prophylactically for desensitising, i.e. inducing tolerance in individuals before development of sepsis in said individual rather than using MALP-2 therapeutically in an ongoing sepsis. It is therefore assumed in the art that lipopeptides are not able to intervene in a developing dysregulated inflammatory disease, like sepsis. That is, there is a common consensus that MALP-2 can not act as a therapeutic agent for treating said type of diseases.

In view of the above, there is an ongoing need for therapeutic agents for treating dysregulated inflammatory diseases, in particular SIRS, CARS, sepsis and MODS.

### Summary of the present invention

The present inventors found that lipopeptides or lipoproteins, like MALP-2, as well as bisacyloxypropylcysteine derivatives are able to intervene with sepsis onset e.g. leading to a significant decrease in mortality in a mouse model for sepsis.

Thus, in a first aspect the present invention relates to a pharmaceutical composition for use in the therapy of dysregulated inflammatory diseases comprising a lipopeptide or lipoprotein or bisacyloxypropylcysteine derivatives of the general formula I as outlined herein.

Particular preferred, the active ingredient for use in the treatment of dysregulated inflammatory disease is a lipopeptide conjugate, a lipoprotein conjugate or bisacyloxypropylcystenyl conjugate, in particular a conjugate wherein the conjugate moiety is polyethyleneglycol.

The pharmaceutical composition is particularly useful in the therapy of SIRS, CARS, sepsis or MODS.

In a further aspect, the present invention relates to a method for the treatment of the dysregulated inflammatory diseases, in particular the dysregulated inflammatory identified above, comprising the step of administering a compound of general formula I as defined herein to an individual in need thereof.

### Brief descriptions of the drawings

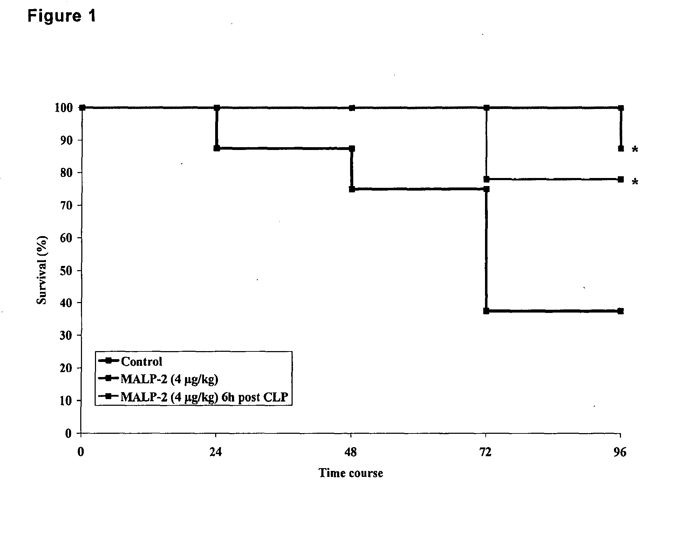

Figure 1: Survival rate (%) following MALP-2 treatment administered parallel to cecal ligation and puncture (CLP) (n=8) and administered 6h after CLP (n=9) in comparison to controls (n=8). Significantly improved survival rates in both MALP-2 groups were found in comparison to the control group (*=p<0.05), the survival rates between MALP-2 treated animals were comparable.

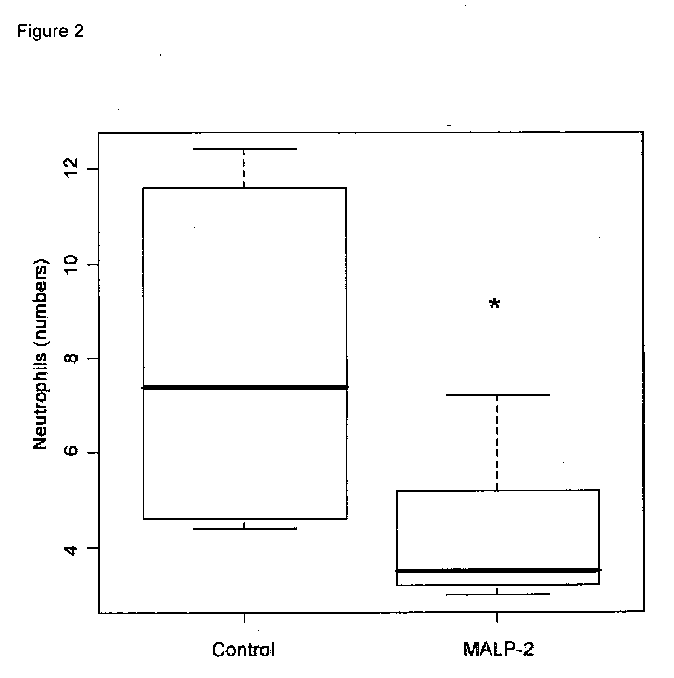

Figure 2: Pulmonary infiltration by PMN in MALP-2 and control animals was determined 4 hours following CLP. The degree of infiltration was determined by the number of Ly-6G positive cells in four representative high power fields. A significantly decreased pulmonary infiltration in MALP-2 treated animals compared to controls could be demonstrated (*=p<0.05).

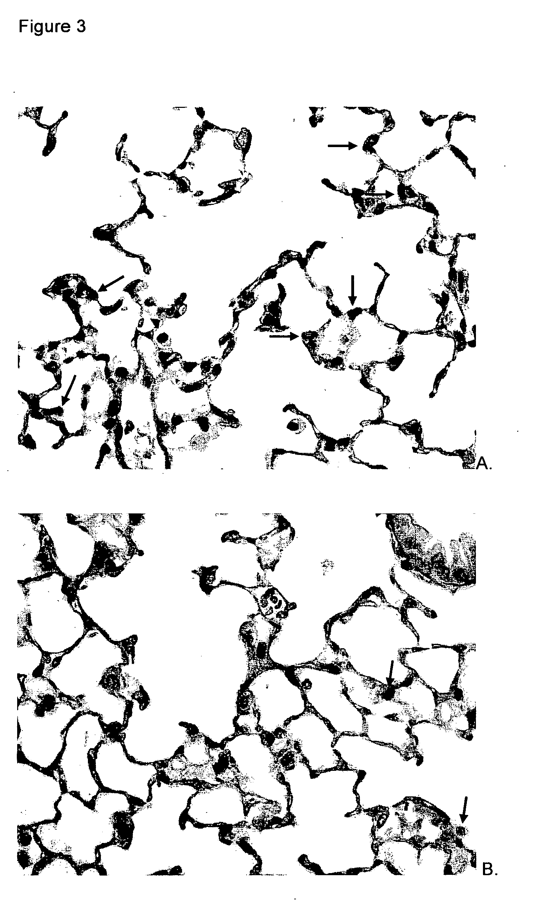

Figure 3: lmmunohistochemical staining of murine neutrophils in paraffin embedded lung tissue specimens with antibody Ly-6G. Staining was visualized using diaminobenzidine.
   A. Stained lung specimen derived from MALP-2 treated animals following CLP;
   B. Stained lung specimen derived from vehicle treated animals following CLP. Black arrows = examples of stained neutrophils.

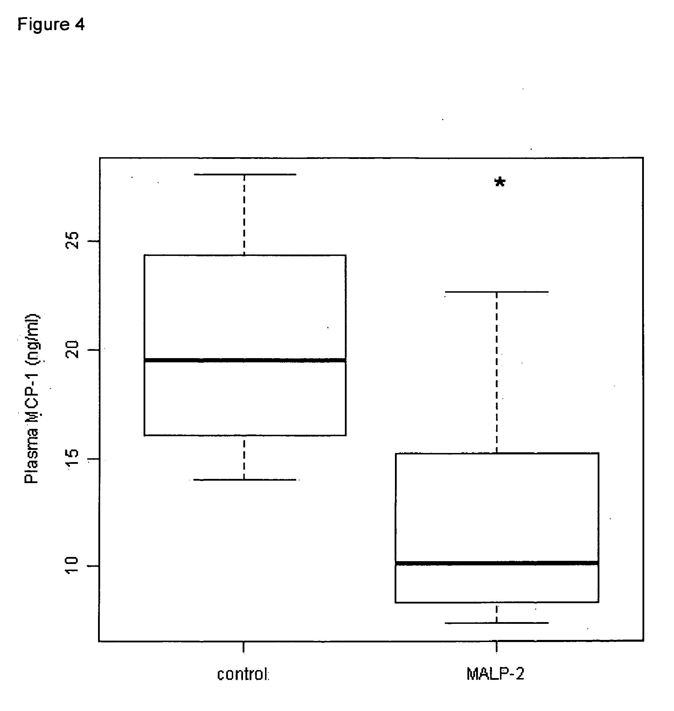

Figure 4: MCP-1 was determined in plasma 4 hours after sepsis induction. In MALP-2 treated animals, we could demonstrate significantly reduced plasma MCP-1 levels compared to control animals. Results are given in ng/ml. (*=p<0.05).

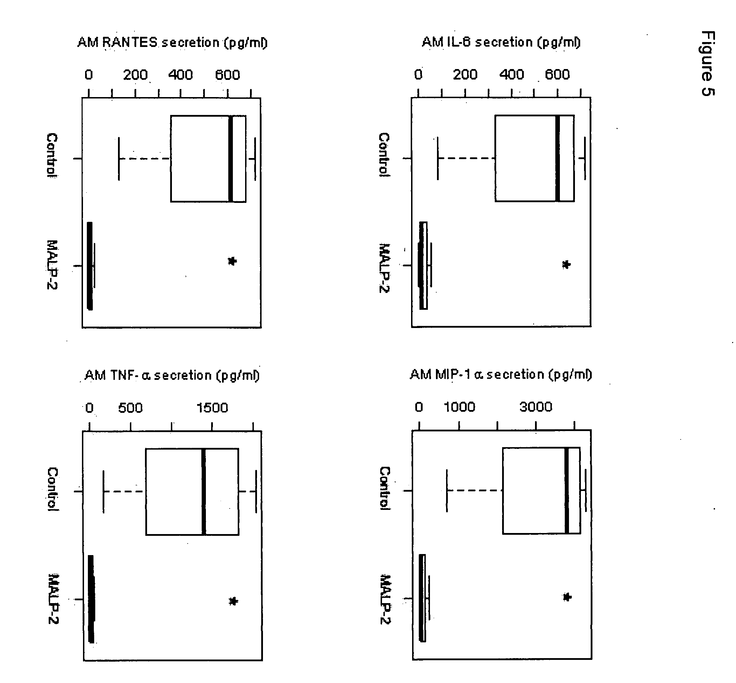

Figure 5: Chemo- and cytokine secretion of isolated alveolar macrophages was analyzed 24 hours after sepsis induction. Cells were incubated for further 24 hours after isolation and levels were determined in cell culture supernatant. Reductions were found for IL-6, MIP-1α, RANTES and TNF-α in comparison to control animals. Results are given in pg/ml. AM= alveolar macrophages. (*=p<0.05).

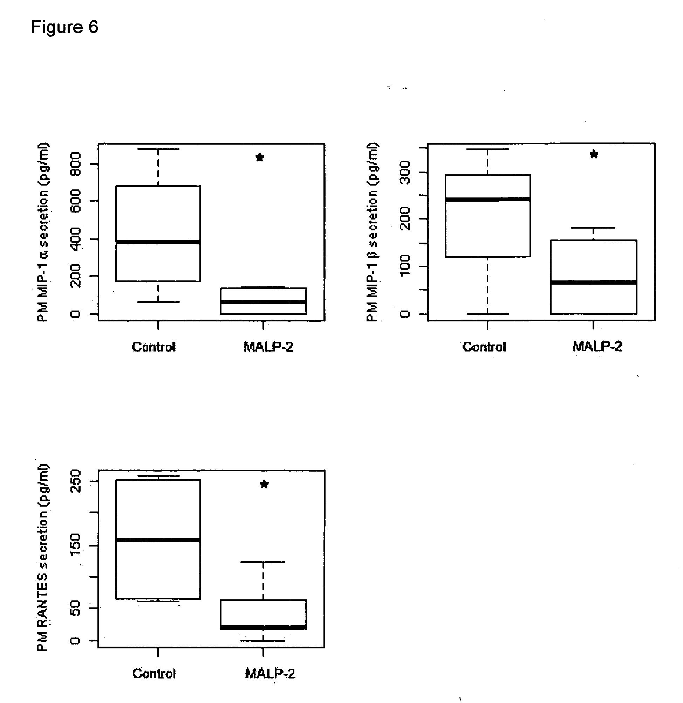

Figure 6: Chemo- and cytokine secretion of isolated peritoneal macrophages was analyzed 24 hours after sepsis induction. Cells were incubated for further 24 hours after isolation and levels were determined in cell culture supernatant. There were reduced levels of MIP-1α, MIP-1β and RANTES after MALP-2 treatment compared to control animals. Results are given in pg/ml. PM= peritoneal macrophages. (*=p<0.05).

### Detailed description of the present invention

In a first aspect, the present invention relates to a pharmaceutical composition for use in the therapy of dysregulated inflammatory diseases comprising as an active ingredient a lipopeptide, lipoprotein or bisacyloxypropylcysteine according to general formula I: 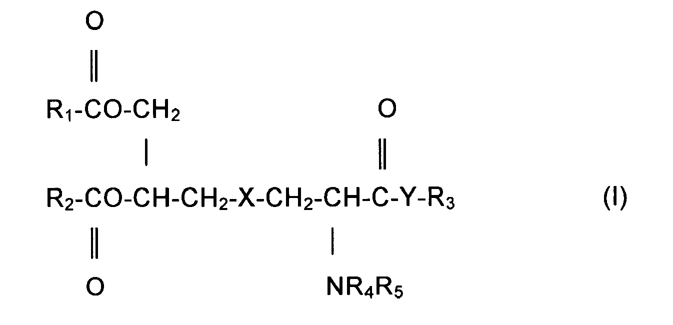 where R₁ and R₂, which may be identical or different, are a C₇ to C₂₅ saturated or unsaturated hydrocarbon moiety, preferably C₇ to C₂₅ alkyl, C₇ to C₂₅ alkenyl and C₇ to C₂₅ alkynyl group, respectively;
R₃ is hydrogen or a water-soluble and physiologically tolerated covalently bonded polymer, in particular, covalently bonded polyethyleneglycol -(CH₂-C₂-O)ₘ-CH₂-CH₂-Z (II),
where Z is OR, N(R)₂, SR, COOR, and R is H, benzyl- or C₁ to C₆ alkyl, where several radicals can be identical or different, a polyoxyethylene-polyoxypropylene-copolymer, a dextran, a sugar, a polyvinylpyrrolidone, an alginate, a pectine or a collagen; m is an integer of 2 to 1000, preferably, 5 to 700, like 10 to 500;
R₄ and R₅ are independently of one another H or C₁ to C₄ alkyl, preferably H or methyl,
X is S, O or CH₂ and
Y is a physiologically tolerated amino acid sequence which consists of one to 25 amino acid residues or Y is NH, O, S or OCO, and the asymmetric carbon atom marked with * has the absolute R-configuration when X is S according to the Cahn-Ingold-Prelog rule.
The present inventors recognized that the above identified compounds of general formula I are useful in treating dysregulated inflammatory diseases, in particular, sepsis and, eventually, decreasing the mortality rate.

According to the present invention, the term "lipopeptide" or "lipoprotein" refers to a compound of general formula I being characterized in having lipophilic moieties, e. g. lipophilic moieties derived or stemming from fatty acids, in particular, alkyl, alkenyl, or alkynyl chains. Said chains may be substituted. Further, said molecules comprises an amino acid derivative moiety, having at least one amino acid residue.

That is, Y is a physiologically tolerated amino acid segment which consist of 1 to 25 amino acid residues.

In a preferred embodiment, Y is a peptide GNNDESNISFKEK (Seq. ID-No. 1), alternatively, Y is GQTDNNSSQSAAPGSGTTNT (Seq. ID-NO. 2).

In an alternative, the molecule of general formula I is a bisacyloxypropyl-cysteine derivative whereby said acyl group is a saturated or unsaturated hydrocarbon moiety, like an alkyl, alkenyl or alkynyl chain, preferably having 7 to 25 C-atoms. Said bisacyloxypropylcysteine derivative is characterized in having a lipophilic moiety as described for the lipopeptide or lipoprotein according to the present invention. The residue Y is selected from NH, O, S or OCO. Preferably, Y is NH or O, particularly preferred O.

The pharmaceutical composition according to the present invention is preferably **characterized in that** the residues R₁ and R₂ which may be identical or different, are independently a C₇ - C₂₅ alkyl, C₇ - C₂₅ alkenyl or C₇ - C₂₅ alkynyl group, preferably a C₈ - C₂₂-alkyl, -alkenyl or -alkynyl group, and the unsaturated positions are preferably in cis configuration, the alkyl-, alkenyl-, and alkynyl residues may be linear, branched or cyclic and may be substituted.

With the term "which may be substituted" is meant a substitution with straight or branched C₁ - C₆ alkyl group or a straight or branched C₁ - C₆ alkoxy group and/or with a halogen, hydroxyl group or carboxyl group.

In another preferred embodiment, the residues R₄ and R₅ are hydrogen or methyl, particular preferred, both residues are hydrogen.

Another preferred embodiment refers to a pharmaceutical composition **characterized in that** the active ingredient according to general formula I is a 2- S-[2,3-bisacyloxy-(2R)-propyl]-L-cysteinyl-peptide or a S-[2,3-bis(acyloxy)-(2R)-propyl]-L-cysteinyl-carboxy-polyethyleneglycol.

Particuarly preferred, the compound to be used according to the present invention is MALP-2 either in natural form isolated from mycoplasma as well as in synthetic form. When prepared synthetically, MALP-2 is characterized in R₁ and R₂ being a palmitoyl group, X is S, R₃, R₄ and R₅ are hydrogen and Y is the peptide of Seq ID. No. 1.

When R₃ is not hydrogen but a water-soluble and physiological tolerated, covalently bonded polymer said "conjugate moiety" R₃, converts the conjugate molecule into a more water-soluble form. The conjugate moiety is **characterized in that** it provides good solubility in hydrophilic solvents, like water, and is not immunogenic.

The conjugate moiety R₃ is preferably a covalently bonded polyethyleneglycol moiety of formula II: -(CH₂-C₂-O)ₘ-CH₂-CH₂-Z (11), where Z is OR, N(R)₂, SR, COOR, and R is H, benzyl- or C₁ to C₆ alkyl, where several radicals can be identical or different, a polyoxyethylene-polyoxypropylene-copolymer, a dextran, a sugar, a polyvinylpyrrolidone, an alginate, a pectine or a collagen, and m is an integer of from 2 to 1000, preferably 5 to 700, like 10 to 500.

In a further preferred embodiment, the pharmaceutical composition according to the present invention contains as an active ingredient a molecule according to general formula I wherein R₃ is a covalently bonded polyethyleneglycol of formula II, and preferably, the polyethyleneglycol has a chain length m of from 5 to 700, preferably of from 10 to 500.

The polyethylene glycol moiety may be a straight or branched structure having polyethyleneglycol units. For example, in case a polyethyleneglycol moiety is present, said moiety may be a branched moiety comprising 4, 6 or 8 arms each having ethyleneglycol subunits.

In another preferred embodiment, the residue X is S. Moreover, it is particularly preferred that Y is the amino acid sequence of Seq. ID No. 1. That is, in a particular preferred embodiment, the active ingredient according to the present invention is MALP-2 or pegylated 2-S-[2,3-bisacyloxy-(2R)-propyl]-L-cysteinyl.

The active ingredients described herein may be in the form of pharmaceutically acceptable non-toxic salts thereof. Salts include acid added salts, such as salts with inorganic acids (e.g. hydrochloric acid, sulphuric acid, nitric acid and phosphoric acid) or with organic acids (e.g. acetic acid, propionic acid, maleic acid, oleic acid, palmitic acid, citric acid, succinic acid, tartaric acid, fumaric acid, glutamic acid, or panthothenic acid.

The pharmaceutical compositions according to the present invention containing a lipopeptide or lipoprotein or bisacyloxypropylcysteine conjugate of general formula I as active ingredient are particulary useful for treating dysregulated informatory diseases, disorders or conditions.

The conjugates according to the present invention, i.e. the compound of general formula I wherein R₃ is a conjugate moiety as definded herein, may be in the form of solvates thereof (e.g., hydrates). In addition, the conjugates may form salts with cationic ions, like metallic ions, in particular alkali or alkaline earth metal ions or NH4+.

Particularly preferred, the pharmaceutical composition is useful in the therapy of systemic inflammatory response syndrome (SIRS), compensatory anti-inflammatory syndrome (CARS), sepsis including severe sepsis and septic shock and multi organ dysfunction syndrome (MODS).

It has been demonstrated that MALP-2 administration following induction of sepsis has a significant effect on the mortality and the release of pro- and anti-inflammatory cytokines by macrophages and activation and infiltration of neutrophilic granulocytes. Although it was known that MALP-2 has beneficial effects in prophylactic treatment by inducing tolerance or desensitisation of the individual, the inventors surprisingly recognized that even after onset of sepsis, MALP-2 as a representative of the lipopeptide and lipoproteins as well as the bisacyloxypropylcysteine derivatives according to the present invention leads to a significant decrease or reduction in inflammation, sepsis or mortality. This decrease of mortality is accompanied by reduced levels of MCP-1, a well known marker molecule for the onset of inflammation and sepsis in plasma, and a reduced chemo- and cytokine liberation by alveolar and peritoneal macrophages. In addition, it has been demonstrated that pulmonary infiltration by neutrophils after MALP-2 administration is decreased. Thus, for the first time, it is possible to provide a therapeutic agent for treating dysregulated inflammatory diseases, disorders or conditions, in particular, SIRS, CARS, sepsis and MODS.

MCP-1 is upregulated in sepsis and correlate with its severity. Recently, Bozza et al., Crit. Care 2007, 11:R49 reconfirmed these findings. In a prospective cohort study, MCP-1 was predictive in both, early and late mortality. Furthermore, it has been demonstrated that a correlation exists between increase of MCP-1 levels and pulmonary infiltration by granulocytes. Here it is demonstrated that after MALP-2 treatment, reduced plasma MCP-1 levels can be detected which are correlated with the reduction of neutrophil infiltration and, in addition, reduced secretion of neutrophil attracting chemokines such as MIP-1 alpha and RANTES from alveolar macrophages.

Hence, the active ingredients of the pharmaceutical composition as defined in general formula I are useful for treating the referenced diseases.

The pharmaceutical composition according to the present invention may further comprise pharmaceutical additives or auxiliary substances, and, preferably, pharmaceutically acceptable carriers, excipients or diluents.

Thus, in a further aspect, the present invention relates to pharmaceutical compositions comprising molecules according to general formula I or salts or solvates thereof as defined herein, in particular, conjugates containing a conjugate moiety or salts or solvates thereof and, optionally, a pharmaceutically acceptable carrier. Such pharmaceutical compositions comprise a therapeutically effective amount of the active ingredients according to the present invention and, optionally, a pharmaceutically acceptable carrier. The pharmaceutical composition may be administered with a physiologically acceptable carrier to a patient, as described herein. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include sodium chloride, water, ethanol and the like. The composition, if desired, can also contain minor amounts of pH buffering agents. These compositions can take the form of solutions. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned conjugates and salts or solvates thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous administration to human beings. Typically, compositions for intravenous administration are solutions in sterile isotonic aqueous buffer. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The term "administered" means administration of a therapeutically effective dose of the aforementioned pharmaceutical composition comprising the conjugates and salts and solvates thereof as defined herein to an individual. "Therapeutically- or pharmaceutically-effective amount" as applied to the compositions of the instant invention refers to the amount of composition sufficient to induce a desired biological result. Said result can be alleviation of the signs, symptoms, or causes of a disease, or any other desired alteration of a biological system. That is, by "therapeutically effective amount" is meant a dose that produces the desired effects. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

In vitro assays may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems.

The administration of the pharmaceutical composition can be done in a variety of ways as discussed above, including, but not limited to, subcutaneously, intravenously, intranodal, intranasally, intraperitoneally, intramuscularly, or intrapulmonary.

The attending physician and clinical factors will determine the dosage regimen

Preferably, the pharmaceutical composition according to the present invention is in the form of a formulation suitable for injection, inhalation or for transnasal administration, in particular, for iv. Administration.

In a further embodiment, the present invention relates to a method for the treatment of dysregulated inflammatory diseases in an individual affected therewith comprising the step of administering an active ingredient as defined by general formula I to an individual in need thereof.

Said dysregulated inflammatory disease, disorder or conditions is in particular SIRS, CARS, sepsis including septic shock and MODS. The compound is administered to the individual in an effective amount of said pharmaceutical composition containing the active ingredient. The pharmaceutical composition may contain additional components, e.g. components like one or more anti-inflammatory molecules.

The methods are applicable to both human therapy and veterinary applications. The compounds described herein having the desired therapeutic activity may be administered in a physiologically acceptable carrier to a patient, as described herein.

The skilled person is well aware of methods for determining the presence of sepsis, namely of an ongoing sepsis as well as manifested sepsis. Further, the physician is aware of means and methods for diagnosing SIRS, etc. For example, a guideline can be found in Bone, R. C., et al. Chest 1992, 101, 1644-1655 which is incorporated by reference herein. Therein, clear definitions of the different forms of diseases and conditions are provided for diagnosing said diseases. Preferably, the compounds according to the present invention are administered when the physician identifies a beginning sepsis with a body temperature above 38,5°C, an intermediate sepsis score and above 15 breaths per minute. In further preferred embodiments, the compounds according to the present invention are administered after diagnosing a likelyhood of sepsis according to the sepsis score as well as after manifestation of sepsis inlcuding determning the presence of bacteria in the blood.

These and other embodiments are disclosed and encompassed by the claims and the description and examples of the present invention.

The invention will be described further in the examples. Said examples are not intended to limit the claimed subject matter but illustrate the invention only further.

### Examples

### Animal care

Maintenance and use of the animals were in accord with the German Animal Welfare Legislation. All experiments were approved by the local institutional animal care and research advisory committee and permitted by the local government of Lower Saxony (Germany). 47 male C57BL/6-mice (Charles River, Germany) with a weight of 20 ± 3 g were used for the study. All animals were handled at room temperature for 14 days before treatment. Animals were maintained under standardized conditions with a controlled environment at 21 ±2°C, relative humidity of 50% and artificial light (14 hours light, 10 hours dark). They received a commercial pellet diet (altromin 1317) and water ad libitum. Analgetic treatment was performed in all animals (200 mg/kg body weight (BW) metamizol-sodium, Novalgin®, Hoechst, Unterschleißheim, Germany) throughout the study.

All surgical procedures were performed after deeply anaesthetizing the animals with isoflurane. The mice were warmed to 36°C using infrared warming lamps after having finished the surgical procedures. All mice received once daily subcutaneous injections of 1 ml sterile ringer's lactate for fluid replacement.

### Group distribution and experimental procedure

Two different treatments were included in the experimental design. The control group received saline as vehicle, the study group was substituted with 4 µg/kg MALP-2 i.p simultaneously to cecal ligation and puncture (CLP). All animals underwent CLP for induction of polymicrobial sepsis. Plasma chemo-and cytokine measurements as well as pulmonary PMN infiltration were determined 4h after CLP (control: n= 6, MALP-2: n= 6), and alveolar as well as peritoneal macrophages were isolated 24h after CLP (control: n= 5, MALP-2: n= 5). Mortality and activity were measured over 96h in the groups that received medication simultaneously to CLP (control: n= 8, MALP-2: n= 8). In another experimental setting, MALP-2 was administered 6h after CLP i.p. (n=9), and mortality was analyzed over 96h. A total amount of 47 mice were used in this study.

The surgical procedure was as follows: the cecum was exposed after a midline laparotomy and ligated distal to the ileocecal valve. Subsequently, the cecum was punctured twice using a 21 G needle and was returned to the abdominal cavity. The model also includes the protrusion of contents of the cecum to assure the presence of bacteria in the peritoneum. The closure of the abdomen was performed with double layer sutures. Data were obtained 4, 24 and 96 hours after surgery. Clinical data concerning mortality and activity were obtained once daily until 96 hours after instrumentation.

### Activity score

As a measure for clinical status, activity of mice was analyzed by using a previously established scoring system, see table 1. Within this score, the spontaneous activity, spontaneous food intake and response to exogenous stimuli was analyzed. The scoring of each animal (from 1= moribund to 6= very active) was independently performed in a blinded fashion by two persons and the mean of both values was recorded.

**Table 1: Activity score**

| **Level** | **Qualitiy** | **Charcateristics of behaviour** |
|---|---|---|
| 6 | Very active | Strong, curious, fast motions |
| 5 | Active | curious, fast, sporadic activity breaks |
| 4 | Reduced active | attentive, frequent activity breaks |
| 3 | Quiet | Disinterested on environment, rare activity, sleepy |
| 2 | Lethargic | no activity, persist in one position, no food uptake |
| 1 | Moribund | no activity, reduced vital functions , death is expected |

### Administration of MALP-2

The lipopeptide MALP-2 was synthesized and purified as described in the art and was kept as stock solution in 33% 2-propanol/water. Shortly before intraperitoneal application, the stock solution was diluted with pyrogen-free NaCl 0.9% (Braun, Melsungen, Germany). 4 µg/kg BW MALP-2 or vehicle were administered i.p in a total volume of 1 ml directly after CLP. Vehicle controls received analogous solutions without MALP-2. In another setting, MALP-2 was administered 6 hours after CLP.

### Collection of blood, cell and organ samples

4h after CLP, mice were exsanguinized by cardiac puncture. Approximately 0.5 mL of heparinized blood could be collected per mouse by this method. The blood was centrifuged at 13000 x g for 5 minutes at room temperature (Eppendorf 3200, Germany). After centrifugation the plasma supernatant was transferred into a fresh tube, snap-frozen and stored at -80 °C until cytokine measurement. Furthermore, a sample of lung tissue was taken, snap-frozen and stored at -80 °C for Ly6G immunohistochemistry. In another experimental setting, lung and peritoneal macrophages were isolated 24 hours after CLP.

### Pulmonary infiltration by granulocytes

lmmunohistochemical staining of infiltrating neutrophils was conducted using anti-mouse Ly-6G (BDBiosciences, Heidelberg) on paraffin embedded 3 µm thick tissue sections. Epitopes were unmasked by microwave treatment in citrate buffer (10mM citric acid, pH 6.0). Subsequently, endogenous peroxidase activity in the sections was inhibited using 3 % hydrogen peroxidase (Merck, Darmstadt, Germany) in methanol (JTBaker, Deventer, the Netherlands). They were incubated with the primary antibody for 1 hour. After washing, an incubation with the secondary antibody (rabbit-anti-rat IgG; horseradishperoxidase conjugated, Dako, Hamburg, Germany) was performed for 1 hour. Reactions were visualized by diaminobenzidine (Dako, Hamburg, Germany). For evaluation of the number of infiltrating neutrophils, sections were blinded and positive cells were counted in 4 representative high power fields per section at 500 x magnification.

### Isolation and cultivation of alveolar macrophages

24 hours after CLP, lungs were dissected and flushed three times with 1 ml phosphate buffered saline (PBS) containing 10 IU heparin (Ratiopharm). After centrifugation for 15 min at 400 x g at 4°C, cells were resuspended in RPMI 1640 (Biochrom) containing 10% heat-inactivated fetal calf serum (FCS) and antibiotics (50 U/ml penicillin, 50 µg/ml streptomycin, both Invitrogen) and 2,5 µg/ml Amphothericin B (Biochrom). 105 cells/ml were plated in a total volume of 200 µl in a 96-well plate, and incubated for 24 hours at 37°C, 95% humidity, and 5% CO2. At the end of the incubation period, the supernatants were removed and stored at -80°C until analysis was performed.

### Isolation und cultivation of peritoneal macrophages

Peritoneal macrophages were isolated by lavage of the peritoneal cavity with 3x5ml PBS containing 10 IU/ml heparin (Ratiopharm) 24 hours after CLP. Thereafter, cells were washed once with PBS and centrifuged at 400xg for 15 minutes at 4°C. The pellet was resuspended in 5ml RPMI (Biochrom) containing 10% FCS and antibiotics (50 U/ml penicillin, 50 µg/ml streptomycin, Invitrogen; 2.5 µg/ml Amphotericin B, Biochrom). 106 cells were plated in a 24-well plate and incubated for 24 hours at 37°C, 95% humidity, and 5% CO2. At the end of the incubation period, the supernatants were removed and stored at -80°C until analysis was performed.

### Cytokine and chemokine quantification

Cytokine and chemokines in plasma and cell culture supernatant were measured with Multiplex and Simplex Kits according to manufacturer's instructions (BenderMed Systems, Vienna, Austria).

### Statistics

Statistical analysis was performed using SPSS (version 15) and R (2.8.1).

The data are summarized as median [interquartile range IQR]. As the data are skewed distributed and variances are heterogeneous, two sample comparisons between groups were performed using two-sided Neubert-Brunner permutation tests. Survival was compared using two-sided Log Rank tests. Probability values less then 0.05 were considered statistically significant.

### RESULTS

### Survival and mortality

Mortality was decreased in MALP-2 group compared to the control group

(figure 2). Mortality in mice treated with 4µg/kgBW showed a total mortality of 12.5%, mortality in the control group was 62.5% (p=0.031).

To investigate the therapeutic effect towards mortality after sepsis onset, MALP-2 was administered 6 hours following CLP. With 22.2%, mortality was decreased compared to vehicle control (p=0.047) (figure 1).

### Activity

Following CLP, all animals demonstrated decreased activity rates as determined according to table 1 after 24 hours and 48 hours. Reduced activity rates were found in the control group from 24 hours to 72 hours following CLP compared to the MALP-2 treated group (p<0.05). However, activity rates after 96 hours were comparable between both groups. This may be explained by drop outs as most of the animals that exhibited low activity scores in the control group had already died at this time point.

### Pulmonary Infiltration

PMN count was reduced in MALP-2 treated animals in comparison to the control group (median [IQR]: 3.5 [3.2-4.9] vs. 7.4 [5-10.8]; p=0.003) (figure 2, figure 3).

### Plasma cytokine and chemokine levels

To investigate the plasma chemokine levels of the treated mice, we analyzed monocyte chemotactic protein (MCP)-1, MCP-3, tumor necrosis factor (TNF)-α interleukin (IL)-6, IL-10, macrophage inflammatory protein (MIP)-1α, MIP-1β, "regulated upon activation, normal T-cell expressed, and presumably secreted" (RANTES) and granulocyte-macrophage colony stimulating factor (GM-CSF). Reductions were found for MCP-1 fozur hours after treatment, median [IQR]: 10125.9 [8617.8-14140.1] vs. 19523.7 [16567.5-23555.6] pg/ml, p=0.031) (figure 4).

### Cytokine and chemokine expression in alveolar macrophages

Reductions for IL-6, MIP-1α, RANTES and TNF-α in supernatants of alveolar macrophages compared to untreated animals were detected (IL-6: 13.2 [8.9-23.9] vs. 598.9 [451.9-646.3], p=0.027; MIP-1α: 38.4 [13.7-99.9] vs. 3785.6 [2858.7-4055], p=0.032; RANTES: 0 [0-6.3] vs. 612.5 [469.5-661] p=0.028; TNF-α: 3.2 [0.1-16.7], p=0.028, all results are given as median [IQR] in pg/ml) (figures 5). For MCP-1, MCP-3, IL-10, GM-CSF and MIP-1β no p-values were computed because in at least one of the two groups all values were equal to 0.

### Cytokine and chemokine expression of peritoneal macrophages

There were reduced levels of MIP-1α, MIP-1β and RANTES after MALP-2 treatment compared to the control group (MIP-1α: 65.2 [0-138.1] vs. 488.9 [271.1-874.5], p=0.01; MIP-1β: 64.3 [0-141.9] vs. 241.7 [241.7-347.4], p=0.03; RANTES: 19.8 [18.9-63.1] vs. 247.5 [68.3-257.4], p=0.031; all results are given as median [IQR] in pg/ml). No differences were determined for IL-6, IL-10, TNF-α, MCP-1, MCP-3 or GM-CSF (p>0.05) (figure 6).

It is demonstrated herein that not only decreased mortality by MALP-2 application at sepsis onset but also by treatment 6 hours after sepsis induction is possible. As clinical parameter, activity rates of MALP-2 treated mice were increased, indicating an overall improvement after CLP and subsequent sepsis. The beneficial effects of MALP-2 treatment are accompanied by reductions of cyto- and chemokine levels in different compartments. Due to the efficacy of late MALP-2 therapy (6 hours after CLP), a mechanism via early immune stimulation and effective clearance rather than via cross-tolerance is suggested. Furthermore, it is postulated that these results are induced by a local influence of MALP-2, primarily affecting peritoneal macrophages. These modulations might result in a systemic influence on circulating cytokines. Finally, an indirect effect mediated by a decreased systemic immune reaction might lead to decreased pulmonary infiltration.

## Claims

1. Pharmaceutical composition for use in the therapy of disregulated inflammatory diseases comprising as an active ingredient, a lipopeptide, lipoprotein or a bisacyloxypropylcysteine derivative of the general formula (I): 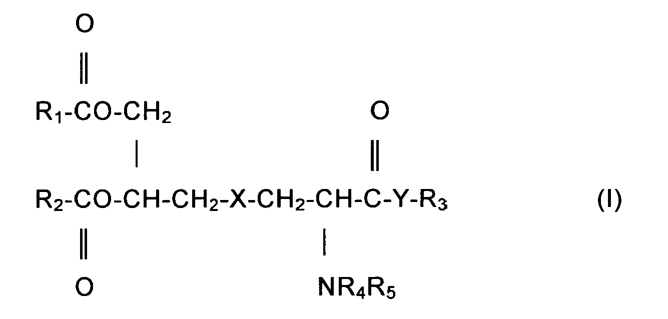 where R₁ and R₂, which may be identical or different, are a C₇ to C₂₅ saturated or unsaturated hydrocarbon moiety, preferably C₇ to C₂₅ alkyl, C₇ to C₂₅ alkenyl and C₇ to C₂₅ alkynyl group, respectively;
R₃ is hydrogen or a water-soluble and physiologically tolerated covalently bonded polymer, in particular, covalently bonded polyethyleneglycol -(CH₂-C₂-O)ₘ-CH₂-CH₂-Z (II),
where Z is OR, N(R)₂, SR, COOR, and R is H, benzyl- or C₁ to C₆ alkyl, where several radicals can be identical or different, a polyoxyethylene-polyoxypropylene-copolymer, a dextran, a sugar, a polyvinylpyrrolidone, an alginate, a pectine or a collagen; m is an integer of 2 to 1000, preferably, 5 to 700, such as 10 to 500;
R₄ and R₅ are independently of one another H or C₁ to C₄ alkyl, preferably H or methyl,
X is S, O or CH₂ and
Y is a physiologically tolerated amino acid sequence which consists of one to 25 amino acid residues or Y is NH, O, S or OCO, and
the asymmetric carbon atom marked with * has the absolute R-configuration when X is S according to the Cahn-Ingold-Prelog rule; or salts or solvates thereof.

2. The pharmaceutical composition according to claim 1, wherein Y consist of 12 to 25 amino acid residues, preferably Y is GNNDESNISFKEK (Seq. ID. No.1) or GQTDNNSSQSAAPGSGTTNT (Seq. ID. No.2).

3. The pharmaceutical composition according to claim 1 or 2, **characterized in, that** the compound according to general formula I is a S-[2,3-bis(acyloxy)-(2R)-propyl]-cysteinyl-peptide or a S-[2,3-bis(acyloxy)-(2R)-propyl]-L-cysteinyl-carboxy-polyethyleneglycol.

4. The pharmaceutical composition according to any one of the preceding claims, wherein R₁ and R₂, which can be identical or different, are C₈ to C₂₂ alkyl, C₈ to C₂₂ alkenyl and/or C₈ to C₂₂ alkynyl group, respectively, and the unsaturated positions are preferably in the cis-configuration, with the alkyl, alkenyl and alkynyl residues being branched or unbranched, cyclic or cycloaklkyl residues which may be substituted.

5. The pharmaceutical composition according to any one of the preceding claims, wherein both R₄ and R₅ are hydrogen.

6. The pharmaceutical composition according to any one of the preceding claims, wherein R₃ is a covalently bonded polyethyleneglycol of formula II and, preferably, the polyethyleneglycol has a chain length m of from 5 to 700, preferably of from 10 to 500.

7. The pharmaceutical composition according to any one of the preceding claims, wherein X is S.

8. The pharmaceutical composition according to any one of the preceding claims wherein the active ingredient is MALP-2.

9. The pharmaceutical composition according to any one of the preceding claims useful in the therapy of systemic inflammatory response syndrome, compensatory anti-inflammatory response syndrome, sepsis or multi organ dysfunction syndrome.

10. The pharmaceutical composition according to claim 9 for treating sepsis in individuals in intensive care units.

11. Pharmaceutical composition according to any one of the preceding claims **characterized in that** it comprises pharmaceutical additives or auxiliary substances, and, preferably, a pharmaceutical acceptable carrier, excipient or diluent.

12. Pharmaceutical composition according to any one of the preceding claims in the form of a formulation suitable for injection, inhalation or for intranasal administration.

13. The pharmaceutical composition according to any one of the preceding claims for use in the treatment of systemic inflammation.

14. Method for the treatment of dysregulated inflammatory diseases comprising the step of administering a compound of general formula I as defined in any one of claims 1 to 8 to an individual in need thereof.


SEQUENCE LISTING

<110>  Helmholtz-Zentrum für Infektionsforschung GmbH
       Medizinische Hochschule Hannover

<120>  Pharmaceutical compositions for treating dysregulated
       inflammatory disease

<130>  3275-022 EP-1

<160>  2

<170>  PatentIn version 3.3

<210>  1
<211>  13
<212>  PRT
<213>  artificial

<220>
<223>  peptide of malp-2

<400>  1

Gly Asn Asn Asp Glu Ser Asn Ile Ser Phe Lys Glu Lys
1               5                   10


<210>  2
<211>  20
<212>  PRT
<213>  artificial

<220>
<223>  peptide

<400>  2

Gly Gln Thr Asp Asn Asn Ser Ser Gln Ser Ala Ala Pro Gly Ser Gly
1               5                   10                  15


Thr Thr Asn Thr
            20